# EUROPEAN PATENT SPECIFICATION

(11) **EP 2 391 497 B1**
(45) Date of publication and mention of the grant of the patent: **04.09.2013**
(21) Application number: 10703165.0
(22) Date of filing: 27.01.2010
(51) Int. Cl.: B29C 63/06, A61F 2/07

(54) **METHOD AND APPARATUS FOR MANUFACTURE OF COVERED STENTS**
VERFAHREN UND VORRICHTUNG ZUR HERSTELLUNG VON UMMANTELTEN STENTS
PROCÉDÉ ET APPAREIL DE FABRICATION DE STENTS RECOUVERTS

(30) Priority: 28.01.2009 US 148011 P
(43) Date of publication of application: 07.12.2011
(73) Proprietor: Abbott Laboratories Vascular Enterprises Limited, Dublin 2 (IE)
(72) Inventor: SEEL, Ismet, 72379 Hechingen (DE)
(74) Representative: Peters, Hajo
(86) International application number: PCT/EP2010/000484
(87) International publication number: WO 2010/086147

(56) References cited:
- WO-A1-95/05132
- US-A- 3 890 182
- US-A- 5 904 804
- US-A- 5 916 264

## Description

### BACKGROUND OF THE DISCLOSURE

The present disclosure relates generally to providing apparatus and methods for covering medical stents. In particular, the present disclosure relates to improved apparatus and methods for applying a cover sheath to a medical stent to provide a stent graft.

### BRIEF SUMMARY

The present disclosure relates to apparatus and methods for applying a cover sheath to a medical stent to provide a stent graft. In particular, the disclosure relates to a semi-automated but optionally hand operatable apparatus to apply a cover to a medical apparatus, such as a stent. Embodiments of the present disclosure may facilitate effectively and/or precisely applying a cover sheath to a medical stent.

Stent grafts are typically used for supporting of vessels, in particular in the case of aneurisms and also in the case of labile or brittle or thrombotic vessels.

Conventional stent grafts typically include a radially expanding stent that is produced, for example, by laser cutting metal tubes and attaching a coating or cover sheath to the stent. Such stent graft is e.g. known from WO 95/05132. The coating and/or cover sheath that may include a fabric and/or foil. The coating may limit blood passage or passage of blood components or deposits through the wall of the stent graft and/or may limit tissue growth through the wall into the interior of the stent graft. Thereby, the vessel wall may have reduced pressure. Embolic formation may be reduced or prevented at the implementation point of the stent graft.

The cover or coating may be of tubular shape with the tube diameter generally corresponding to the dimensions of the stent to which the cover will be attached. In some embodiments, the cover may be made from a flat sheath of covering and/or graft material which may be wrapped around the stent. In some embodiments, the wrapping can be performed in a single layer, a dual layer, multiple layers in thickness, or combinations thereof depending on the graft material and/or the specific needs for the resulting stent graft.

As the graft material sheath is wrapped over the stent precision may necessitate (i) precisely placing the graft material on the stent, (ii) tightly fitting the stent and the graft material, (iii) avoiding folds and/or crinkles, (iv) other considerations, or (v) combinations thereof. Further, the placement of the graft material sheath may be time-consuming and/or may usually be performed by hand-operation. Also, as stent grafts may have dimensions as small as about 5 mm in length and/or about I mm in diameter, the handling of the sheath and/or stent may be very cumbersome.

It may therefore be desirable to provide apparatus and methods for applying a cover sheath to a medical stent to provide a stent graft.

This object is achieved by the apparatus defined in claim 1 and by the method defined in claim 5. According to the present disclosure, an embodiment of an apparatus for applying a graft material to a stent is provided. The apparatus includes,
(a) a mandrel adapted to receive the stent within the apparatus,
(b) a forming roller lying axially parallel to and in contact with the stent, whereby rotation of the forming roller rotates the stent about its longitudinal axis,
(c) mechanical means for applying a sheath of graft material to the stent as the stent is rotated about its axis,
(d) mechanical means for synchronising the rotation of the stent with the means for applying the graft material, the stent receiving mandrel may be a hollow tube provided with small apertures in the region of the receiving area of the stent to allow a vacuum to be applied in order to temporarily affix the graft material sheath to the stent once the graft material came into contact with the stent, or combinations thereof.

The means for applying the sheath of graft material to the stent may include a pressure roller lying axially parallel to the stent and/or arranged to press the sheet slot against the stent. The sheath of graft material may be in the form of pre-cut sheath that can be fed manually into the pressure roller.

The means for synchronising the rotation of the stent with the means for applying the sheath of graft material to the stent may be provided by intermediate gearing means in operative cooperation with the forming roller. In one embodiment, the stent on the stent receiving mandrel itself may operate as means for synchronizing the rotation of the stent with the means for applying the sheath of graft material to the stent.

. The stents used as the frame for the stent graft for use by the apparatus of the disclosure may be balloon expandable and/or self expandable. Suitable materials for forming these stents may includes stainless steel, cobalt-chromium alloys, or Nitinol in case of self-expandable stents. Other metals, alloys, polymers, composites, plastics, other materials, or combinations thereof may be possible.

The cover or coating can be affixed to the stent in many different ways including gluing, sewing, folding around the stent, other ways, or combinations thereof. In one embodiment, as depicted in United States Patent No. 5,916,264, stent graft may include two coaxially arranged radial extending stents, and a flexible, stretchable material layer, such as a cover sheath, may be provided between both stents, and thus may be clamped between the two coaxially arranged stents.

Cover materials suitable for use by an apparatus according to the present disclosure may include flexible sheaths and/or foils of woven and/or knitted materials, such as but not limited to polyethylene terephthalate and/or of porous polytetrafluoroethylene (hereinafter PTFE). Grafts of biological origin may alsobe used, like biological materials with autologous or homologous veins or arteries, these being typically fixed human umbilical or bovine arteries. Other materials may be used to form the sheath, foils, webs, or other structures forming the cover material. For instance, other metals, alloys, polymers, plastics, composites, other materials, or combinations thereof may be used.

A PTFE foil may be used that has a fibril structure and is unidirectionally expandable and thus can undergo radial expansion where the typical PTFE graft material is usually co-axially placed over the stent when the stent is implanted into the vessel by, for example, balloon expansion.

To produce the sandwich construction stent graft depicted in United States Patent No. 5,916,264 a sheath of, for example, PTFE foil may be wrapped around a first stent resulting in a two to three layered PTFE cover around the first stent. Subsequently, the second stent may be disposed over the first stent and the cover material and may be affixed to the first stent to result in the sandwich stent graft.

These and other objects and features of the present disclosure will become more fully apparent from the following description and appended claims, or may be learned by the practice of the disclosure as set forth hereinafter.

### BRIEF DESCRIPTION OF THE DRAWINGS

To further clarify the above and other advantages and features of the present disclosure, a more particular description of the disclosure will be rendered by reference to specific embodiments thereof which are illustrated in the appended drawings. It is appreciated that these drawings depict only illustrated embodiments of the disclosure and are therefore not to be considered limiting of its scope. The disclosure will be described and explained with additional specificity and detail through the use of the accompanying drawings in which:

FIG. 1 is a partial cutaway perspective view of an apparatus according to the disclosure.

FIG. 2 is a rear perspective view of an apparatus according to the disclosure.

### DETAILED DESCRIPTION

The present disclosure is directed to apparatus and methods for applying a cover sheath to a medical stent to provide a stent graft. More specifically, at least one embodiment of the present disclosure is directed to apparatus and methods for wrapping a sheath of graft material over a stent, thereby ensuring precise location of the cover material and a tight fit of the material to the stent.

An embodiment of the present disclosure is further directed to a apparatus for applying a graft material to a stent, the apparatus including,
(a) a mandrel adapted to receive the stent within the apparatus,
(b) a forming roller lying axially parallel to and in contact with the stent, whereby rotation of the forming roller rotates the stent about its longitudinal axis,
(c) mechanical means for applying a sheath of graft material to the stent as the stent is rotated about its axis, and
(d) mechanical means for synchronising the rotation of the stent with the means for applying the graft material, the stent receiving mandrel being a hollow tube provided with small apertures in the region of the receiving area of the stent to allow a vacuum to be applied in order to temporarily affix the graft material sheath to the stent once the graft material came into contact with the stent, or combinations thereof

In one embodiment, the means for applying the sheath of graft material to the stent may include a pressure roller lying axially parallel to the stent and arranged to press the sheath of graft material against the stent. The sheath of graft material may be in the form of pre-cut sheath that can be fed manually into the pressure roller.

In a further embodiment, the means for synchronising the rotation of the stent with the means for applying the sheath of graft material to the stent may be provided by intermediate gearing means in operative cooperation with the forming roller.

In one embodiment, the stent on the stent receiving mandrel itself may operate as means for synchronising the rotation of the stent with the means for applying the sheath of graft material to the stent by contacting both means.

Another embodiment of the present disclosure is further directed to a method for manufacturing a stent graft, the method including:
a) providing an apparatus having a hollow mandrel adapted to receive a stent, the mandrel having apertures in the region where the stent is imposed on the mandrel;
b) providing a stent;
c) imposing the stent on the mandrel;
d) applying a vacuum to the inside of the mandrel;
e) providing a sheath of a graft material of a size suitable to cover the stent in the desired area;
f) contacting a first portion of the graft material sheath with the outer surface of the stent mounted on the mandrel, thereby creating a tight temporary fit between the first end of the graft material and the stent by the drawing force of the vacuum; and
g) wrapping the graft material around the outer surface of the stent by rotation of the mandrel with the stent imposed thereon, or combinations thereof.

In one embodiment, the method further includes affixing the wrapped graft material to the stent. In a further embodiment, the wrapped graft material may be affixed to the stent by the superimposition of a second stent over the first stent and the graft material. In further embodiments, the graft material can also be affixed to the stent by, for example but without limitation, gluing, sewing, folding, or combinations thereof around the stent, and/or by imposing cuffs, rings, zigzag formed crowns, or combinations thereof over the stent and/or graft material ends to clamp the ends of the graft material onto the stent.

In a further embodiment, the method for wrapping a sheath of graft material over a stent may include releasing the vacuum and/or removing the stent graft from the mandrel.

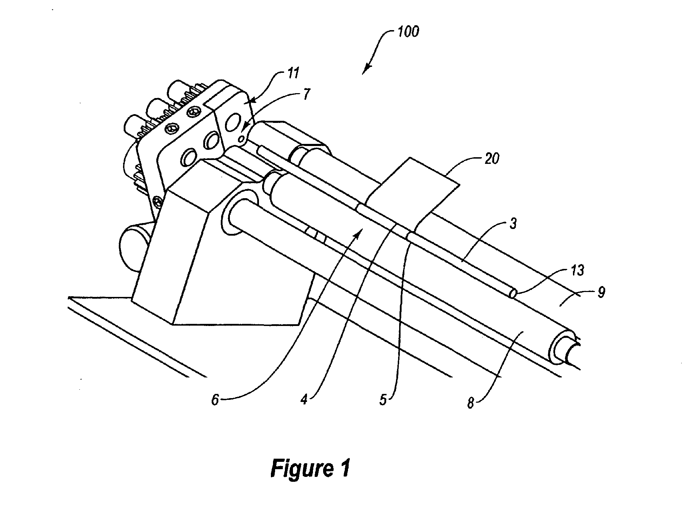

Referring now to Figure 1, there is shown an embodiment of an apparatus 100 according to the present disclosure. A mandrel 3 may be adapted to receive a stent 4 and/or may be made from a hollow tube with apertures 5 in the region of the receiving area 6 of the stent. The mandrel 3 may be loosely positioned in the apparatus in Figure 1. The mandrel may be air tightly affixed to the connection port 7 in the apparatus which may be connected to a vacuum pump (not shown) for generation of a vacuum or at least a very low pressure in the mandrel. On the mandrels end opposite to the connection port, the mandrel may be closed and/or sealed with a plug 13.

A forming roller 8 may be axially parallel to and/or in contact with the stent 4 on the mandrel 3. Rotation of the forming roller 8 may rotate the stent 4 about its longitudinal axis. Mechanical means for applying a sheath of graft material to the stent as the stent is rotated about its axis may include a second roller 9 that may be parallel to the stent 4 on the mandrel 3. In one embodiment, and as shown in Figure 1, mechanical means for synchronising the rotation of the stent with the means for applying the graft material are provided. An example of means for synchronising the rotation of the stent with the means for applying the graft material may include gear configuration 11. In another embodiment, these means may be provided by the close contact between each of the two rollers 8, 9 and the stent 4 on the mandrel 3. It will be understood that the two rollers 8, 9 may be arranged parallel to each other without touching each other.

As further shown in Figure 1, a sheath of graft material 20 of a predetermined size suitable to cover the stent 4 to be covered may be inserted for operation of the apparatus into the inter-space between the second roller 9 and the stent 4. Air flowing through the apertures 5 in the mandrel 3 may be effected by the vacuum drawn inside the mandrel 3. The graft material 20 may be releasably attached to and/or adducted to the stent 4 affixed to the hollow mandrel 3. This adduction may facilitate a more precise location and/or tighter fit of the graft material 20 to the outer surface of the stent 4.

Activation of the apparatus 100 may initiate the rotation of the forming roller 8. The rotation of the forming roller 8 may rotate the stent 4 about its longitudinal axis. The mechanical means for applying the sheath of graft material 20 to the stent or roller 9 may be rotated by the forming roller 8. The second roller 9 may be rotated and may feed the graft material 20 onto the rotating stent 4, thus wrapping the graft material 20 around the outer surface of the stent 4.

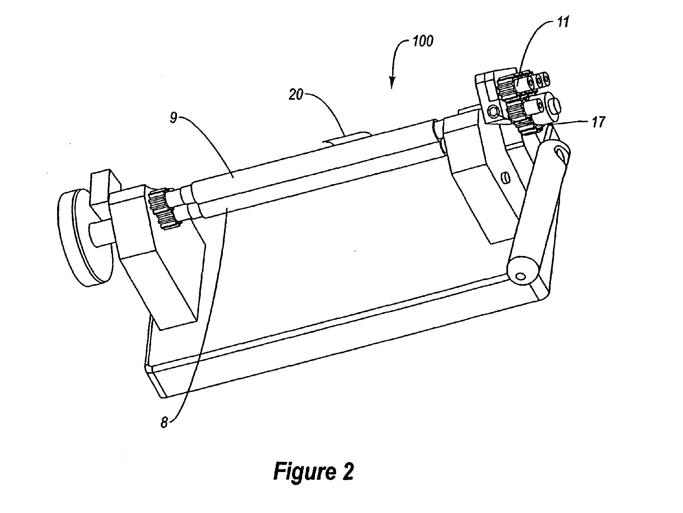

Figure 2 shows the apparatus from the back allowing a closer look at the mechanical means for synchronizing the rotation of the stent with the means for applying the graft material, such as gear configuration 11. Also the connection port 17 for connection of a vacuum pump is shown.

The described embodiments are to be considered in all respects only as illustrative and not restrictive.

## Claims

1. An apparatus (100) for applying a graft material to a stent (4), the apparatus comprising:
(a) a mandrel (3) adapted to receive the sten (4) within the apparatus, the stent receiving mandrel (3) being a hollow tube provided with small apertures in the region of the receiving area of the stent to allow a vacuum to be applied in order to temporarily affix a graft material sheath (20) to the stent,
(b) a forming roller (8) lying axially parallel to and in contact with the stent (4), whereby rotation of the forming rolle (8) rotates the stent about its longitudinal axis,
(c) mechanical means (9) for applying a sheath of graft material (20) to the stent (4) as the stent is rotated about its axis, and
(d) mechanical means (11) for synchronising the rotation of the sten (4) with the means (9) for applying the graft material.

2. The apparatus according to claim 1, wherein the means (9) for applying the sheath of graft material to the stent includes a pressure roller lying axially parallel to the stent and arranged to press the sheath of graft material (20) against the stent (4).

3. The apparatus according to claim 1 or 2 wherein the mechanical means (11) for synchronising the rotation of the stent (4) with the means for applying the sheath of graft material to the stent is provided by intermediate gearing means in operative cooperation with the forming roller (9).

4. The apparatus according to claim 1 or 2 wherein the stent (4) on the stent receiving mandrel (3) itself operates as means for synchronising the rotation of the stent with the means for applying the sheath of graft material to the stent by contacting both means.

5. A method for manufacturing a stent graft comprising:
a) providing an apparatus (100) comprising a hollow mandrel (3) adapted to receive a stent (4) the mandrel having apertures in the region where the stent is imposed on the mandrel;
b) providing a sten (4);
c) imposing the sten on the mandrel (3);
d) applying a vacuum to the inside of the mandrel (3);
e) providing a sheath of a graft material (20) of a size suitable to cover the stent (4) in the desired area;
f) contacting a first portion of the graft material sheath (20) with the outer surface of the the stent (4) mounted on the mandrel (3), Thereby creating a tight temporary fit between the first end of the graft material and the stent by the drawing force of the vacuum; and
g) wrapping the graft material (20) around the outer surface of the stent (4) by rotation of the mandrel (3) with the stent imposed thereon.

6. The method according to claim 5 further comprising affixing the wrapped graft material (20) to the stent (4)

7. The method according to claim 6 wherein the wrapped graft material is affixed to the stent (4) by the superimposition of a second stent over the first stent and the graft material.

8. The method according to any of claims 5 to 7 further comprising a final step of releasing the vacuum and removing the stent graft from the mandrel (3);

## Patentansprüche

1. Vorrichtung (100) zum Aufbringen eines Transplantatmaterials auf einen Stent (4), Folgendes aufweisend:
(a) einen Spanndorn (3), der zur Aufnahme des Stents (4) innerhalb der Vorrichtung eingerichtet ist, wobei der den Stent aufnehmende Spanndorn (3) ein in dem Aufnahmebereich des Stents mit schmalen Öffnungen versehenes Hohlrohr ist, um zu ermöglichen, dass zum vorübergehenden Befestigen einer Transplantatmaterialhülle (20) an dem Stent ein Vakuum angelegt wird,
(b) eine Formwalze (8), die achsenparallel zu dem Stent (4) liegt und mit ihm in Kontakt steht, wobei die Drehung der Formwalze (8) den Stent um seine Längsachse dreht,
(c) mechanische Mittel (9) zum Aufbringen einer Hülle aus Transplantatmaterial (20) auf den Stent (4), wenn der Stent um seine Achse gedreht wird, und
(d) mechanische Mittel (11) zum Synchronisieren der Drehung des Stents (4) mit den Mitteln (9) zum Aufbringen des Transplantatmaterials.

2. Vorrichtung gemäß Anspruch 1, wobei die Mittel (9) zum Aufbringen der Hülle aus Transplantatmaterial auf den Stent eine Anpresswalze umfassen, die achsenparallel zum Stent (4) liegt und eingerichtet ist, die Hülle aus Transplantatmaterial (20) gegen den Stent (4) zu pressen.

3. Vorrichtung gemäß Anspruch 1 oder 2, wobei die mechanischen Mittel (11) zur Synchronisation der Drehung des Stents (4) mit den Mitteln zum Aufbringen der Hülle aus Transplantatmaterial auf den Stent durch Zwischengetriebemittel, die sich mit der Formwalze (9) in Arbeitsverbindung befinden, bereitgestellt sind.

4. Vorrichtung gemäß Anspruch 1 oder 2, wobei der Stent (4) auf dem den Stent aufnehmenden Spanndorn (3) selber als Mittel zur Synchronisation der Drehung des Stents mit den Mitteln zum Aufbringen der Hülle aus Transplantatmaterial auf den Stent durch den Kontakt mit beiden Mitteln wirkt.

5. Verfahren zur Herstellung eines Stenttransplantats, umfassend:
a) Bereitstellen einer Vorrichtung (100), die einen hohlen Spanndorn (3) umfasst, der zur Aufnahme eines Stents (4) eingerichtet ist, wobei der Spanndorn in dem Bereich Öffnungen aufweist, in dem der Stent über den Spanndorn gestülpt ist;
b) Bereitstellen eines Stents (4);
c) Überstülpen des Stents (4) über den Spanndorn (3);
d) Anlegen eines Vakuums an der Innenseite des Spanndorns (3);
e) Bereitstellen einer Hülle aus einem Transplantatmaterial (20) in einer Größe, die geeignet ist, den Stent (4) in dem gewünschten Bereich zu bedecken;
f) Inkontaktbringen eines ersten Abschnitts der Transplantatmaterialhülle (20) mit der Außenfläche des Stents (4), der auf dem Spanndorn (3) befestigt ist, wodurch eine vorübergehende, eng anliegende Passung zwischen dem ersten Ende des Transplantatmaterials und dem Stent durch die ziehende Kraft des Vakuums erzeugt wird; und
g) Wickeln des Transplantatmaterials (20) um die Außenfläche des Stents (4) durch Drehung des Spanndorns (3) mit dem darüber gestülpten Stent.

6. Verfahren gemäß Anspruch 5, ferner die Befestigung des umwickelten Transplantatmaterials (20) an dem Stent (4) umfassend.

7. Verfahren gemäß Anspruch 6, wobei das umwickelte Transplantatmaterial durch das Überstülpen eines zweiten Stents über den ersten Stent und das Transplantatmaterial befestigt wird.

8. Verfahren gemäß einem der Ansprüche 5 bis 7, ferner in einem letzten Schritt das Auflösen des Vakuums und das Abziehen des Stenttransplantats von dem Spanndorn (3) umfassend.

## Revendications

1. Appareil (100) d'application d'un matériau de greffe sur un stent (4), cet appareil comprenant .
(a) un mandrin (3) apte à recevoir le stent (4) dans l'appareil, le mandrin (3) recevant le stent (4) étant un tube creux pourvu de petits orifices dans la zone recevant le stent afin de permettre d'appliquer un vide afin de fixer temporairement une gaine de matériau de greffe (20) sur le stent,
(b) un rouleau formeur (8) reposant axialement parallèlement au stent (4) et en contact avec lui, la rotation du rouleau formeur (8) faisant tourner le stent autour de son axe longitudinal,
(c) un moyen mécanique (9) pour appliquer une gaine de matériau de greffe (20) sur le stent (4) pendant que le stent tourne autour de son axe et
(d) un moyen mécanique (11) pour synchroniser la rotation du stent (4) avec le moyen (9) d'application du matériau de greffe.

2. Appareil selon la revendication 1, dans lequel le moyen (9) d'application de la gaine de matériau de greffe comprend un rouleau compresseur reposant axialement parallèlement au stent et conçu pour comprimer la gaine de matériau de greffe (20) contre le stent (4).

3. Appareil selon la revendication 1 ou 2, dans lequel le moyen mécanique (11) de synchronisation de la rotation du stent (4) avec le moyen d'application de la gaine de matériau de greffe sur le stent est créé par des moyens de transmission intermédiaires coopérant opérationnellement avec le rouleau formeur (9).

4. Appareil selon la revendication 1 ou 2, dans lequel le stent (4) posé sur le mandrin récepteur de stent (3) fait lui-même office de moyen de synchronisation de la rotation du stent avec le moyen d'application de la gaine de matériau de greffe sur le stent en mettant les deux moyens en contact.

5. Procédé de fabrication d'une greffe de stent comprenant les étapes consistant à :
(a) se procurer un appareil (100) comprenant un mandrin creux (3) apte à recevoir un stent (4), le mandrin étant pourvu d'orifices dans la zone où le stent est posé sur le mandrin,
(b) se procurer un stent (4) ;
(c) poser le stent (4) sur le mandrin (3) ;
(d) appliquer un vide à l'intérieur du mandrin (3) ;
(e) se procurer une gaine de matériau de greffe (20) d'une taille adaptée pour couvrir le stent (4) dans la zone souhaitée ;
(f) mettre en contact une première partie de la gaine de matériau de greffe (20) avec la surface extérieure du stent (4) monté sur le mandrin (3) en créant ainsi un ajustement temporaire hermétique entre la première extrémité du matériau de greffe et le stent par la force de traction du vide ; et
(g) enrouler le matériau de stent (20) autour de la surface extérieure du stent (4) par rotation du mandrin (3) sur lequel le stent est posé.

6. Procédé selon la revendication 5, comprenant en outre la fixation du matériau de greffe enroulé (20) au stent (4).

7. Procédé selon la revendication 6, dans lequel le matériau de greffe enroulé est fixé au stent (4) par superposition d'un second stent au premier stent et au matériau de greffe.

8. Procédé selon l'un quelconque des revendications 5 à 7, comprenant en outre une étape finale de relâchement du vide et de retrait de la greffe de stent du mandrin (3) .
